# EUROPÄISCHE PATENTSCHRIFT

(11) **EP 0 735 923 B1**
(45) Veröffentlichungstag und Bekanntmachung des Hinweises auf die Patenterteilung: **16.06.1999**
(21) Anmeldenummer: 95903761.5
(22) Anmeldetag: 23.12.1994
(51) Int. Cl.: B03C 5/00, B03C 5/02, G01N 33/483, G01N 33/487

(54) **ADHÄSIONSSTEUERBARE ULTRAMINIATURISIERTE OBERFLÄCHENSTRUKTUR**
ULTRA-MINIATURISED SURFACE STRUCTURES WITH CONTROLLABLE ADHESION
STRUCTURE DE SURFACE ULTRA-MINIATURISEE A ADHESION REGLABLE

(30) Priorität: 23.12.1993 DE 4344351; 14.01.1994 DE 4400955
(43) Veröffentlichungstag der Anmeldung: 09.10.1996
(73) Patentinhaber: FRAUNHOFER-GESELLSCHAFT ZUR FÖRDERUNG DER ANGEWANDTEN FORSCHUNG E.V., 80636 München (DE)
(72) Erfinder: FUHR, Günter, D-13127 Berlin (DE); VOIGT, Andreas, D-13051 Berlin (DE); HAGEDORN, Rolf, D-13027 Berlin (DE); LISEC, Thomas, D-10319 Berlin (DE); MÜLLER, Torsten, D-12439 Berlin (DE); WAGNER, Bernd, D-14199 Berlin (DE)
(74) Vertreter: Leonhard, Frank Reimund, Dipl.-Ing.
(86) Internationale Anmeldenummer: DE9401530
(87) Internationale Veröffentlichungsnummer: WO9517258

(56) Entgegenhaltungen:
- WO-A-91/11262
- DE-A- 4 127 405
- ELECTROPHORESIS, Bd. 13,Nr. 1/2, Januar 1992 - Februar 1992 DEERFIELD BEACH, FL, US, Seiten 49-54, XP 000430628 R. HAGEDORN, ET AL. 'TRAVELING-WAVE DIELECTROPHORESIS OF MICROPARTICLES'
- JOURNAL OF PHYSICS D APPLIED PHYSICS., Bd. 26,Nr. 9, 14.September 1993 LETCHWORTH GB, Seiten 1528-1535, XP 000430867 Y. HUANG, ET AL. 'ELECTROKINETIC BEHAVIOUR OF COLLOIDAL PARTICLES IN TRAVELLING ELECTRIC FIELDS: STUDIES USING YEAST CELLS'

## Beschreibung

Die Erfindung betrifft eine leitfähige Oberflächenstruktur, um suspendierte mikroskopische Partikel und Zellen zu beeinflussen. Sie betrifft auch die Verwendung dieser Struktur zur Steuerung der Adhäsionseigenschaften der Partikel und Zellen.

In der Medizintechnik, der Biokompatibilitätsforschung, insbesondere bei der Herstellung transplantierbarer Materialien, aber auch in der biologisch-pharmakologischen Forschung wird seit langem nach Oberflächen gesucht, die einerseits abstoßend auf in physiologischen und technischen Lösungen befindliche Partikeln und Zellen wirken, andererseits in bestimmten Fällen die Adhäsion fördern (BOGRAND, P. (ed.), Physicl Basis of Cell-Cell-Adhesion, CRC Press, Inc., Boca Raton, Florida, 1988. CURTIS, A.S.G., PITTS, J.D. (eds.), Cell Adhesion and Motility, Cambridge University Press, Cambridge, 1980. GRINELL, F., Int. Rev. Cytol, 53:65-144, 1978. LEE, L.H., Recent Advances in Adhesion, Gordon & Breach, London, 1973. OTTEWILL, R.H., ROCHESTER, C.H., SMITH, A.L. (eds.), Adsorption from Solution, Academic Press, London. PERELSON, A.S., DeLISI, Ch., WIEGEL, F.W., Cell Surface Dynamics, Concepts and Models, Marcel Dekker, Inc., New York, Basel, 1984). In der Regel wird diese "Oberflächenmodifizierung" über Hydrophilisierung oder Hydrophobisierung, über die Ankopplung geladener molekularer Gruppen oder durch lokale Ankopplung hochspezifischer Bindungsstellen (z.B. Antikörper) erreicht. Nachteilig an diesen Oberflächenmodifizierungen ist die geringe Reichweite in die Partikelsuspension (in der Regel wenige Å), die sehr unterschiedliche Langzeitstabilität, sowie die nicht vorhandene Steuerbarkeit der Effekte.

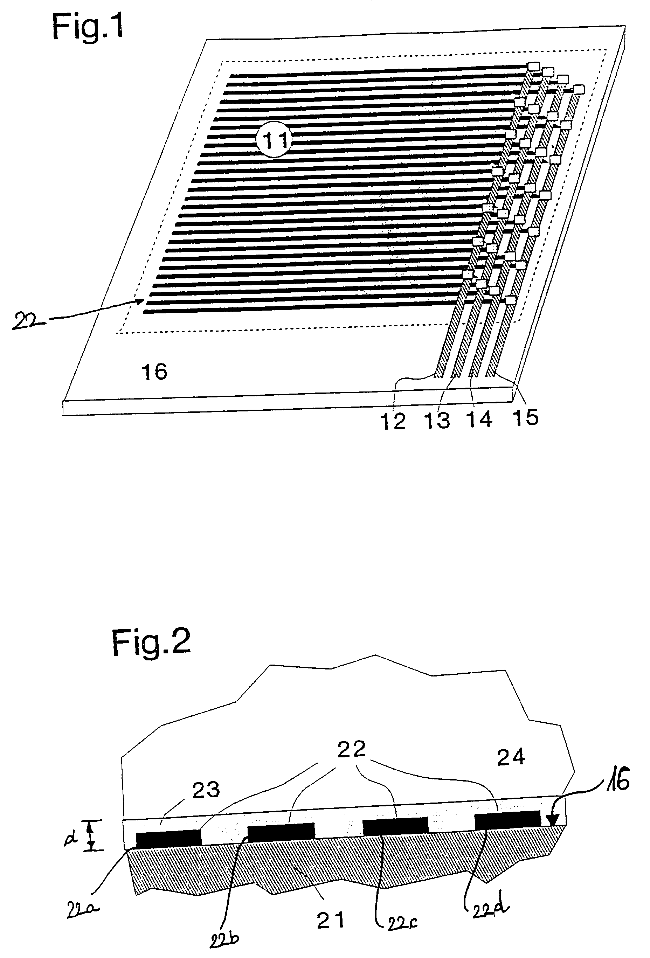

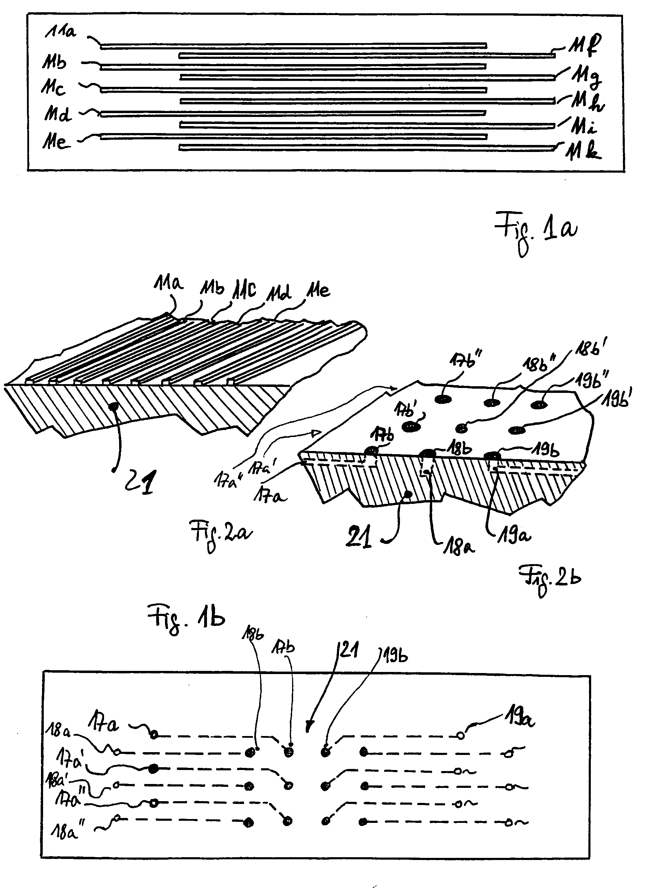

Daß elektrische Felder über Elektroden in eine Partikel- oder Zellsuspension ausgekoppelt werden können und über Polarisation der Teilchen Moleküle und Zellen zu den Elektroden hin- oder wegdrücken können, wurde ausführlich von POHL 1978 (POHL, H.P., Dielectrophoresis, Cambridge Press, Cambridge 1978) untersucht und in Patenten, wie US 4,390,403, fixiert. Diese als Dielektrophorese bezeichneten Kräfte können sowohl **anziehend** (positive Dielektrophorese) als auch **abstoßend** (negative Dielektrophorese) wirken. Das Phänomen wird sowohl zur Sammlung von Schmutzpartikeln in makroskopischen Filtern als auch zur Sammlung und Separation von Zellen und Mikropartikeln in Mikrostrukturen genutzt, allerdings bisher aus folgenden Gründen nur begrenzt nutzbar:
(i) Elektroden wurden im makroskopischen Bereich verwendet und, bis zu einigen Mikrometern miniaturisiert, auch auf planaren Oberflächen erzeugt. Die in die Flüssigkeit entkoppelten elektrischen Hochfrequenzfelder durchdringen dann mit nahezu gleicher Feldstärke die gesamte Zelle, woraus eine **hohe Belastung** der Objekte (Zellen und Partikel) resultiert und sehr hohe Anregungsspannungen erforderlich sind (einige V bis zu einigen 100V).
(ii) Die Elektroden sind immer noch so groß, daß sich Zellen auf ihnen **ablagern können**, obwohl das Feld angeschaltet ist, da sie auf der breiteren Elektrode keine Nachbarelektroden mehr spüren, wodurch der angestrebte Effekt zunichte gemacht wird. Dieses Problem entsteht z.B. in der **WO 91/11262** (P&B Sciences Limited), wo zwar ein Elektrodenarray benutzt wird, das kammerförmig (vgl. die dortige Figur 1B) sein kann, nicht aber dessen Größe und Abmessung an die zu beeinflussenden Partikel anpaßt. Diese WO befaßt sich vielmehr ausschweifend mit den Größen und Formen von "non-uniform-Magnetfeldern" und deren Einfluß auf chemische Reaktionen **zwischen** den Zellen.
(iii) Mit den Elektroden in Kontakt gekommene oder direkt von ihnen angezogene Zellen oder Partikel **verändern sich** auf Grund der Metall-Zelloberflächenreaktion irreversibel.

Hochfrequente Wanderwellen, erzeugt über elektrische Signale, wurden von MELCHER zum Pumpen von Ölen genutzt (MELCHER, J.R., The physics of fluids, 9:1548-1555, 1966). Ende der 80er, Anfang der 90er Jahre konnte dieses Prinzip auch in Mikrokanälen mittels halbleitertechnologisch gefertigter Elektrodenstrukturen umgesetzt werden. Das Prinzip beruhte auf der Stabilisierung eines Temperaturgradienten und der Erzeugung phasenverschobener Raumladungen. Auch hier lag die Elektrodenbreite bei einigen 10µm (FUHR, G. et al., MEMS 92, Proceedings, 1992).

Daß auch Partikel und Zellen mittels wandernder elektrischer Felder selektiv bewegt werden können, wurde von MASUDA (MASUDA, S., IEEE Transaction on Industry Applications, 24, 217-222, 1988) gezeigt und 1991 auf hochfrequente Wanderwellen ausgedehnt (FUHR, G. et al., MEMS 91, Proceedings, 259-264,1991). Ziel dieser planaren Anordnung war die Bewegung einzelner Zellen in Mikrokanalsystemen mit dem Ziel der **Zellseparation**, wie sie in **WO 91/3850 oder DE-A 41 27 405** (Fraunhofer-Gesellschaft) näher erläutert ist. Dort wird eine im Aufbau aber nicht in der Größenordnung mit dem Anspruch 1 vergleichbare Elektrodenstruktur beschrieben, welche Struktur in Richtung des Wanderfeldes hinsichtlich Ausdehnung und Abstand in der Größenordnung der biologischen Teilchen, also im Bereich weniger µm liegt (vgl. dort Spalte 2, Zeile 45 ff). Diese Struktur vermag nur Transportfunktion auf die Teilchen auszuüben.

Der Erfindung liegt **die Aufgabe zugrunde**, eine Oberfläche in ihrem Adhäsionsverhalten, insbesondere für suspendierte Zellen und Makromoleküle elektrisch steuerbar zu verändern.

Das wird mit Anspruch 1 erreicht. Die abhängigen Ansprüche konkretisieren und erweitern den Gedanken der elektrisch steuerbaren Adhäsionswirkung eines - insbesondere dielektrisch abgedeckten - Submikrometer-Elektroden-Areals. Das Areal kann als Streifenfläche oder als Punktfläche ausgebildet sein.

Da die Feldstärke mit dem reziproken Abstand der Elektroden und proportional zur angelegten Spannungsdifferenz zunimmt, ergibt sich erst im anspruchsgemäßen Submikrometerbereich (Anspruch 17) die Möglichkeit mit geringen Amplituden (100mV bis 1V) die erforderlichen Feldstärkegradienten auf einer Seite der Partikeln und Zellen zu erzeugen, die mit der **DE-A 41 27 405** noch nicht zur Verfügung gestellt werden konnten.

Die in die Flüssigkeit eingekoppelten Feldstärkegradienten werden mit schmaler werdenden Elektroden auch schneller mit dem Abstand von der Oberfläche abfallen, da sich die Anteile im oberflächenfernen Flüssigkeitsbereich überlagern und kompensieren. Dieser Umstand vermindert die elektrische und thermische Belastung der Partikeln außerordentlich.

Erfindungsgemäß läßt sich dieser Effekt über die Applizierung wandernder elektrischer Oberflächenwellen, vornehmlich im Hochfrequenzbereich (kHz - MHz) verstärken, so daß unter den oben geschilderten Randbedingungen eine elektrische Steuerung der Adhäsionseigenschaften der Oberfläche erfolgen kann und die Teilchen erst dann in den elektrischen Einflußbereich der Oberfläche gelangen, wenn sie der Oberfläche sehr nahe kommen.

Erfindungsgemäße Verwendungen der erwähnten Struktur sind von den Ansprüchen 22 bis 24 erfaßt. Anspruch 21 betont die Ausbildung der blanken Elektroden-Areale. Die jeweiligen Spannungsbereiche sind im Anspruch 16 umschrieben.

Mittels einer im Submikronbereich (unterhalb von 1µm) strukturierten planaren Oberfläche können die mikroskopischen und submikroskopischen Partikel oder Zellen (im folgenden: "Teilchen") elektrisch gesteuert an der Isolierschicht angelagert oder abgestoßen werden, ohne sie hohen Belastungen auszusetzen, ohne sie chemisch zu verändern und ohne mit dauerhaften nicht ablösbaren Ablagerungen konfrontiert zu werden. Die Elektrodenareale erhalten eine neue Eigenschaft kraft ihrer Dimensionierung. Die Elektroden können dielektrisch bedeckt oder blank sein (Ansprüche 1,21). Sind sie blank, werden den streifenförmigen oder punktförmigen Elektroden deutlich geringere Spannungen zugeführt, um die Verluste klein zu halten (Anspruch 16). Höhere Spannungen (im Voltbereich) erlauben die dielektrisch bedeckten Elektroden. Im Subminiatubereich können so lokal kräftige Felder erzeugt werden, ohne daß die Zellen vollumfänglich mit Kräften beaufschlagt werden. Die Elektrodendimensionierung erlaubt das unsymmetrische (einseitige) Beaufschlagen der Zellen, da Ihre Größe oberhalb des Subminiaturbereichs liegt.

Mit den Elektrodensystemen der Erfindung kann mit physiologischen (leitfähigen) und sogar mit hochleitfähigen Lösungen gearbeitet werden. Dieser Umstand erweitert die medizinisch-biologische Anwendung elektrischer Feldtechniken um einen Anwendungsbereich, der bisher für nicht funktionsfähig erachtet wurde.

Der Erfindung liegt dabei die Erkenntnis zugrunde, extrem schmale Elektrodenbänder oder -knöpfe in sich wiederholender Folge auf eine Oberfläche (Substrat) aufzubringen, die so schmal sind, daß im günstigsten Falle mehrere Elektrodenbahnen oder ein Umfeld von eng beieinander liegenden Punkten dem typischen Durchmesser der Teilchen entsprechen.

Unter Submikronbereich versteht die Erfindung einen Bereich, der bei etwa 1µm beginnt und nach unten unbegrenzt ist, allerdings derzeit durch zur Verfügung stehende Fertigungstechniken auf Bereiche um 100nm bis 500nm beschränkt bleibt. Gleichwohl haben Experimente gezeigt, daß die patentgemäßen Wirkungen umso deutlicher zu Tage treten wenn die Abmessungen weiter verringert werden.

Ein direkter Kontakt der Partikel mit den Elektroden wird durch eine aufgelagerte, insbesondere biokompatible (Anspruch 17), dielektrische Schicht verhindert, deren Materialeigenschaften so gewählt werden, daß ein elektrisches Feld mit einer noch ausreichenden Feldstärke in den oberflächennahen Flüssigkeitsraum ausgekoppelt werden kann.

Die aufgebrachten erfindungsgemäßen Schichten koppeln einerseits das wandernde elektrische Feld in geeigneter Weise in den oberflächennahen Flüssigkeitsraum aus und verhindern andererseits elektrolytische Prozesse, so daß sie in physiologischen Lösungen hoher Leitfähigkeit eingesetzt werden können (Anspruch 22).

Die Isolier-Schichten (Merkmalsgruppe b des Anspruches 1) sind solche Schichten, die die wässrige oder leitfähige Lösung mit den darin suspendierten Teilchen von den Elektroden trennt, demnach "isoliert". Diese Isolierung ist sowohl mechanisch gemeint, sie ist ebenso elektrisch gemeint, es kann auch eine Kombination der beiden vorerwähnten Isoliermöglichkeiten sein. Günstig ist ein hoher Dielektrizitäts-Koeffizient, zwingend erforderlich ist er allerdings nicht.

Bevorzugte Ausführungs**beispiele** der Erfindung werden nachfolgend anhand der Figuren näher erläutert.
**Figur 1 und Figur 3** sind jeweils perspektivische Ansichten der mikrostrukturierten Oberfläche mit elektrisch leitenden Elektrodenstreifen 22,42,44 und aufgelagerten Isolier-Schichten 23,45,43.
**Figur 2 und Figur 4** sind Schnittbilder der in Figur 1 und Figur 3 dargestellten Strukturen.
**Figuren 1a und 1b** sind jeweils Ansichten einer mikrostrukturierten Oberfläche mit elektrisch leitenden Elektrodenstreifen 11 (11a bis 11k) bzw. 17b, 18b, 19b, **ohne** aufgelagerte Isolier-Schichten.
**Figuren 2a und 2b** sind die zugehörigen perspektivischen Schnittbilder der in Figur 1a und 1b dargestellten Strukturen, einmal mit aufliegenden Streifenelektroden 11a, 11b, ... und einmal mit punktförmigen Elektroden 17b, 17b', 17b'' und im Substrat eingebetteten Zuleitungen 17a, 18a, 19a.

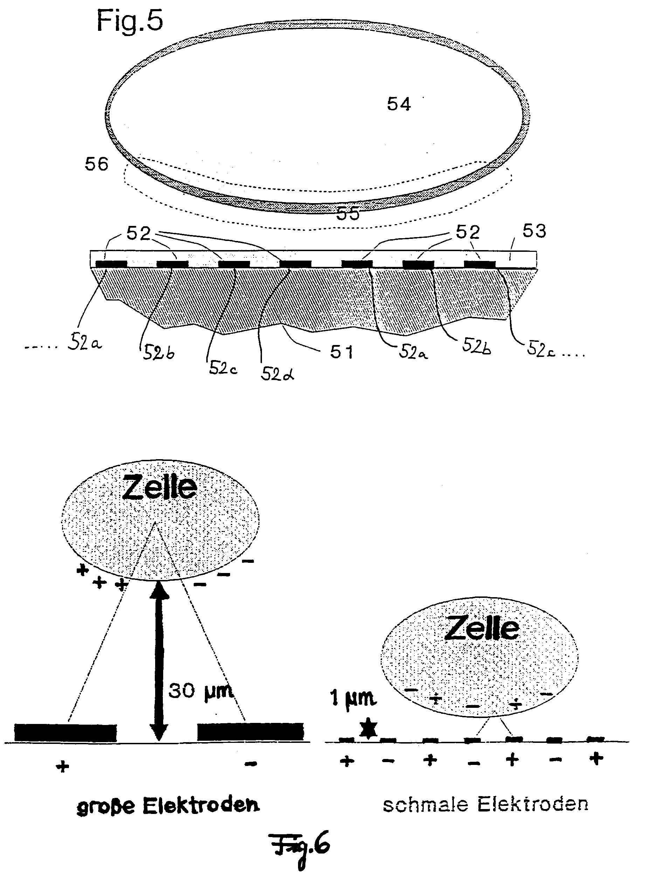

**Figur 5** dient der Erläuterung der unsymmetrischen Kraftwirkung auf ein zelluläres Objekt 54, dessen Dimension größer ist als die Struktur der Elektroden.

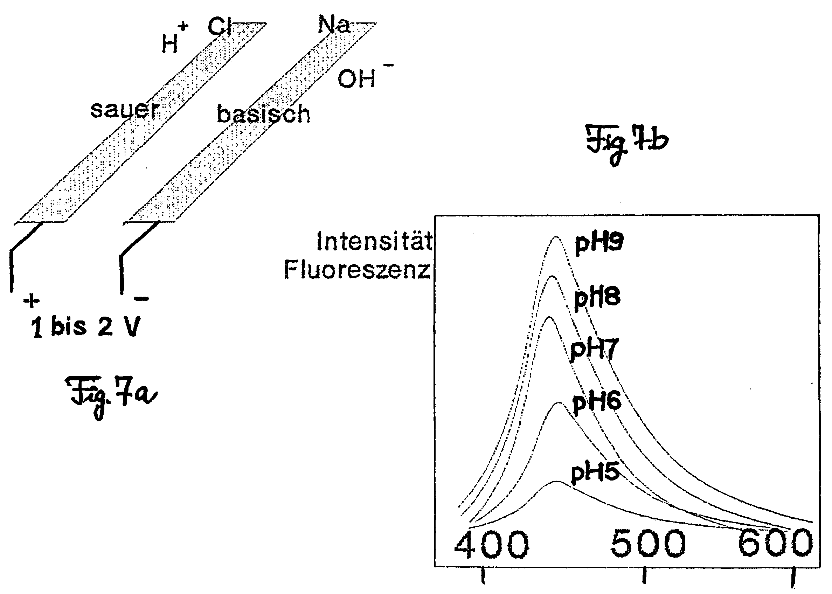

**Figur 6** veranschaulicht in zwei Bildern die zellschonende Wirkung von Oberflächenstrukturen im 1µm-Bereich.
**Figur 7a und Figur 7b** sind Darstellungen, wie mit potentialmäßig gesteuerten Elektroden Säure und Base (auch Anode und Kathode) erzeugbar sind um dem pH-Gradienten von Figur 7b im µm-Bereich zu stabilisieren.

**Figur 1 und Figur 2** zeigen eine in ihrem Adhäsionsverhalten (bezüglich Teilchen in Suspension) elektrisch steuerbare planare Oberfläche mit einer Elektrodenstreifenfläche 11. Die Elektroden 22 sind über 4 Zuleitungen 12,13,14 und 15 zu Gruppen von jeweils 4 Elektroden zusammengefaßt und periodisch in dieser Gruppenkonfiguration (a,b,c,d ...) ansteuerbar. In gleicher Weise können Gruppen aus jeweils 3 oder auch mehr Elektroden gebildet werden. Auf diese Weise ist es möglich, in ihrer Richtung festlegbare elektrische Wanderwellen zu erzeugen.

Im angegebenen Beispiel wird dies erreicht, indem vier um jeweils ein Viertel in der Phase verschobene periodische Signale an die Zuleitungen 12,13,14 und 15 angelegt werden, so daß sich über der Elektroden-Streifenfläche 11 ein mit einer vorgebbaren Geschwindigkeit ausbildendes Wanderfeld erzeugt wird, das die Teilchen in der Suspension schonend bewegt.

Die den Elektroden aufgelagerte Isolier-Schicht 23 koppelt die wandernden Felder dielektrisch in die darüber befindliche Flüssigkeit 24 aus und kann biokompatibel sein.

Die Oberflächenwellen werden umso kräftiger ausgekoppelt, je höher die relative Dielektrizitätskonstante und je dünner die Isolier-Schicht 23 gewählt werden. Typische Dicken für die beschriebenen Anwendungen sind einige 10nm bis zu einigen Mikrometern ("Sub-Mikrometerbereich"). Die aufgelagerte Schicht kann jedoch auch durch mono-, bi- und multimolekulare Schichten ersetzt oder ergänzt werden. Mit diesen Schichten können die zellspezifischen biokompatiblen Eigenschaften wesentlich unterstützt werden. Als Materialien kommen beispielsweise Polyuretan, Teflon, Metall- und Halbleiteroxide oder -isolatoren (SiO₂,SiC,Si₃N₄) in Frage. Die molekularen Schichten können Lipide, Detergentien, Polymere oder ähnliches sein.

Über diese Isolier-Schichten 23 können die Oberflächen zusätzlich lateral in ihren Adhäsionseigenschaften strukturiert und prädeterminiert werden.

Die Steuerung der Adhäsionseigenschaften erfolgt über das Anlegen der hochfrequenten Signale mit Frequenz, gegeseitigem Phasenbezug und Amplitude. Im Beispiel wandern die elektrischen Oberflächenwellen jeweils in eine Richtung. Durch veränderte Beschaltung der Elektroden über die Zuführungen 12,13,14 und 15 können gegenläufige, stehende und alternierende Wellen erzielt werden. Die so gesteuerte Einheit kann auf feste oder flexible Substrate 21 (mit der Oberfläche 16) makroskopisch flächenbedeckend aufgebracht werden.

Diese Strukturen können auch in aufgerollter Form die Wände von Schläuchen oder Reaktionsräumen (treatment cells) bilden.

Die beschriebenen Strukturen werden mit bekannten Herstellungstechniken der Halbleitertechnologie, z.B. in Elektronenstrahl-Lithographie und bekannten Abscheide- und Ätzverfahren erzeugt.

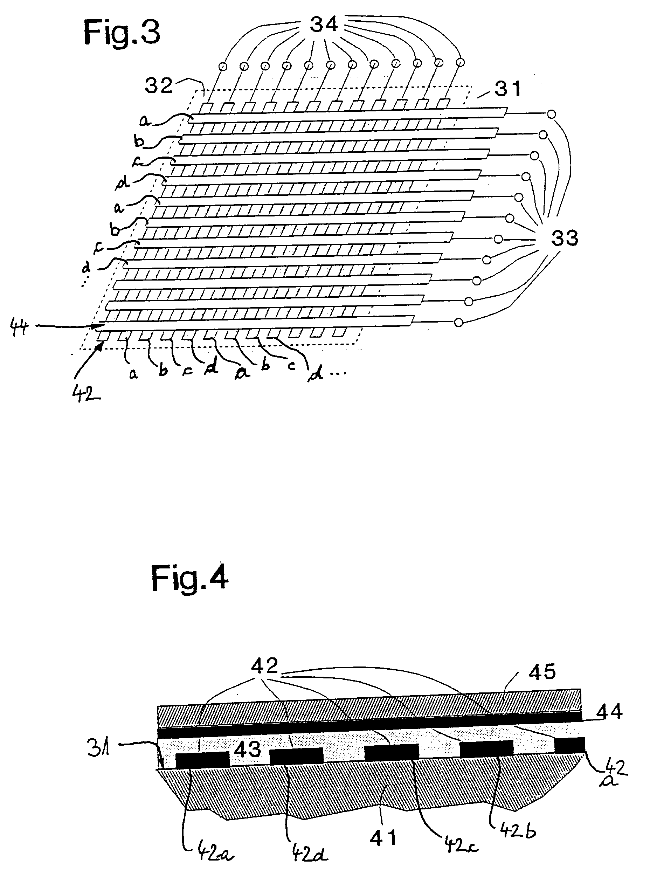

**Figur 3 und Figur 4** zeigen eine Oberflächenstruktur bei der zwei zueinander dielektrisch isolierte Elektroden-Streifenflächen 42,44 um 90° gedreht angeordnet sind. Die gesamten Elektrodenstreifen sind mit Isolier-Deckschichten 31,32,45 passiviert, über die die wandernden Oberflächenwellen beider Elektrodenebenen in die Flüssigkeit ausgekoppelt werden können. Je nach Anwendungszweck können die Einzelelektroden entweder in Gruppen zusammengefaßt und miteinander verknüpft bzw. einzeln angeschlossen sein. Der Anschluß erfolgt über Zuführungen 33,34, die zu den Elektrodenstreifen oder -gruppen führen.

Der Verlauf der Streifen-Elektroden (Figuren 1, 1a, 1b) muß nicht gerade sein, sondern kann in gebogenen, gezackten, mäanderförmigen oder spiraligen Formen auf der Oberfläche 41 verlaufen. Damit lassen sich zusätzlich zu den Adhäsions-Eigenschaften über dem Elektrodenareal befindliche Teilchen sammeln und an bestimmten Orten des Oberflächenarrays ablegen bzw. von diesem entfernen. Muster können gesteuert erzeugt werden.

**Figur 1a und 2a** zeigen eine im Adhäsionsverhalten (bezüglich Teilchen in Suspension) elektrisch steuerbare Oberfläche mit einer Elektrodenstreifenfläche 11a, 11b, 11c, (kurz: 11), der **keine** dielektrische Schicht überlagert ist. Es handelt sich also um blanke Elektroden. Die an sie anlegbare Spannung liegt im unteren Voltbereich - je kleiner die Streifenbreite und der Streifenabstand, desto kleiner auch die Spannung.

**Figur 1b und Figur 2b** zeigen eine im Adhäsionsverhalten (bezüglich Teilchen in Suspension) elektrisch steuerbare Oberfläche mit Elektroden-Punkten 17b, 17b', 17b'', 18b, 18b', 18b'' und 19b, 19b' und 19b''. Die "Elektroden-Buttons" sind von **keiner** dielektrischen Schicht bedeckt. Ihr gegenseitiger Abstand liegt im Bereich von 100nm bis 1µm. Die an sie angelegten Spannungen liegen im Bereich der vorgenannten zu Figur 1a. Die Zuleitungen zu den Elektroden-Punkten sind im Substrat 21 verlegt. Damit sind die Zuleitungen 17a, 17a', 17a'', 18a... und 19a,... isoliert von der Suspension; elektrische Verluste können vermieden werden.

**Figur 5** zeigt die schematischen Größenverhältnisse, wie sie bei der Verwendung tierischer Zellsuspensionen 56 mit Zellgrößen 54 von einigen Mikrometern auftreten und stellt den günstigsten Fall für physiologisch stark leitfähige Zellsuspensionen dar. Es ist ersichtlich, daß die submikrometerbreiten Elektroden 52 den Einfluß der beschriebenen Oberflächenstrukturen auf den oberflächennahen Raum 55 beschränken. Dadurch ist die belastungsarme Beeinflussung des Zell- und Partikel-Adhäsionsverhaltens gegeben.

Es ist davon auszugehen, daß der Oberflächen-Wanderwelleneinfluß in Lösung nur 2 bis 5 Elektrodenbreiten reicht. Damit ist ein nach der Partikelgröße unterschiedlicher Strukturierungsgrad definiert.

Jedes der gezeigten Ausführungsbeispiele kann geometrisch variiert und in Hybridstrukturen eingeordnet werden.Ebenfalls kann die erfindungsgemäße Oberflächenstruktur auch zur Freihaltung von Mikroelementen genutzt werden, die als Sensor eingesetzt werden.

Die **Figur 6** veranschaulicht die Wirkung, die mit den zuvor erläuterten Subminiatur-Strukturen gegenüber einer Zelle in der Größenordnung von einigen 10µm erhalten wird. Während große Elektroden, die einen Abstand von Mitte zu Mitte in der Größenordnung von 30µm haben, die Zelle stark großflächig mit positiven und negativen Influenzladungen belasten, so daß in der Zelle ein erhebliches Potentialgefälle entsteht, wird dies mit den im rechten Halbbild gezeigten Subminiatur-Elektroden nicht mehr geschehen, da sich hier die unterschiedlichen Ladungen an der Oberfläche der Zelle mit einer Periode wiederholen, die der Periode der Subminiatur-Elektrodenstreifen etwa entspricht, so daß nur geringes Potentialgefälle entsteht und sich ein gleichförmig alternierendes Spannungsgefälle an der Oberfläche der Zelle aufbaut. Dieses ist für die Zelle besonders schonend und belastet sie nur wenig, während die von den schmalen Elektrodenstreifen auf die Zelle aufgebrachte Kraft gleichwohl erheblich ist.

Mit dieser Wirkung und den Strukturen gemäß den Figuren 1, 3 und 5 werden also schonende Feldkraft-Abschirmungen, sogenannte "Field Force Shields" in begrenztem Bereich oberhalb der Subminiatur-Elektrodenstruktur erreicht.

Ein Beispiel für Dimensionierung und Werkstoffe, mit dem beispielsweise die **Figur 5** implementiert wurde, ist folgendermaßen:
Gold-Elektrodenstreifen 52 haben eine Breite von 500nm. Die Lücke zwischen den Elektrodenstreifen 52a,52b,52c,52d ... beträgt jeweils 500nm. Die Elektrodenhöhe ist - abweichend von der rechteckigen Struktur der Figur 5 - etwa auch 500nm. Die Elektrodenstreifen-Struktur wurde mit Elektronenstrahl-Lithographie erzeugt. Das Substrat 51 ist Silizium. Die Abdeckschicht 53 ist aus Glas. Ebenfalls aus Glas ist der Zwischenraum zwischen jeweils zwei Elektroden-Streifen. Das eingesetzte Feld (die eingesetzte Spannung) hat eine Frequenz von 1MHz und eine Spannung von 1.5Vₚₚ. Die verwendete Erythrozyten-Suspension hat eine Leitfähigkeit von 1.2S/m. Auch nach mehr als einer Stunde blieb die Oberflächenstruktur des beschriebenen Beispiels ohne jede Ablagerung von Erythrozyten.

Das Beispiel und die Darstellung der verschiedenen Subminiatur-Strukturen zeigt die Möglichkeit auf, daß biokompatible Oberflächen geschaffen werden können, bei denen verhindert werden kann, daß sich die Teilchen ablagern, so daß Trombosen vermieden werden können. Daneben bietet sich die Anwendungsmöglichkeit der Sensorik, bei der verhindert werden kann, daß sich Partikel auf den Sensorflächen ablagern, womit Sensoren entstehen, die eine hohe Lebensdauer haben.

Weitere Anwendungen der beschriebenen Strukturen sind die Implantationstechnik und der Aufbau von Optiken.

Nicht unerwähnt bleiben soll die anhand der **Figuren 7a und 7b** erläuterte Anwendungsmöglichkeit des im µm-Bereich stabilisierten pH-Gradienten. Mit ihm bietet sich die Möglichkeit der Sichtbarmachung von Elektrodendefekten, mit ihm können chemische Mikro-Reaktionssysteme aufgebaut werden, mit ihm können pharmakologische Testsysteme entstehen, schließlich können biologische/medizinische Zellkulturen erforscht werden.

Die Elektrodenstruktur gemäß der Figur 7a erlaubt die potentialmäßige Steuerung und die Erzeugung von Säure und Base auch Kathode und Anode. Dies gelingt allein mit der elektrischen Ansteuerung der im µm-Bereich geschaffenen Elektroden-Struktur. Die Einzelelektroden werden asymmetrisch mit geringen Spannungen im Bereich zwischen 1V bis 2V gepulst. Abhängig von dem Elektrodenabstand (Figur 7b) entstehen unterschiedliche pH-Werte, mit denen ein Gradient oberhalb der Elektroden-Struktur geschaffen werden kann. Der pH-Gradient kann über einen pH-abhängigen Fluoreszenzmarker sichtbar gemacht werden.

## Patentansprüche

1. Adhäsionssteuerbare Oberflächenstruktur mit Elektroden (11;52;22;42,44;17b,18b,19b) auf einem Substrat (21,41,51) zur Verwendung mit einer wässrigen oder leitfähigen Lösung und darin suspendierten mikroskopischen und submikroskopischen Partikeln oder Zellen (Teilchen), bei welcher Oberflächenstruktur
(a) die Elektroden (11;52;22;42,44;17b,18b,19b) eine Breite und einen Abstand im Submicronbereich haben und mit zueinander phasenverschobenen oder gepulsten hochfrequenten periodischen Signalen in Gruppen beaufschlagbar (12 bis 15;34,33; 17a,18a,19a) sind,
(b) um die daraus resultierenden wandernden und/oder stehenden elektrischen Oberflächenwellen - insbesondere über die Elektroden bedeckende Isolier-Schichten (23;53;43,45) - in die oberflächennahe wässrige oder leitfähige Lösung auszukoppeln und das Adhäsionsverhalten in einer Richtung etwa senkrecht zur Substratfläche zu steuern.

2. Oberflächenstruktur nach Anspruch 1, **dadurch gekennzeichnet,** daß die über jeweils mindestens drei Elektroden (22a,22b,22c,22d) erzeugten wandernden elektrischen Oberflächenwellen in ihrer Wanderrichtung periodisch umgeschaltet werden und/oder aufeinander zu bzw. voneinander weg laufen.

3. Oberflächenstruktur nach Anspruch 1 oder 2, **dadurch gekennzeichnet**, daß die über jeweils mindestens drei Elektroden (22a,22b,22c) erzeugten wandernden elektrischen Oberflächenwellen unterschiedliche Wandergeschwindigkeiten dadurch besitzen, daß die Elektroden (22;42,44;52) der Gruppen mit in der Frequenz verschiedenen periodischen Signalen angesteuert werden.

4. Oberflächenstruktur nach einem der erwähnten Ansprüche, **dadurch gekennzeichnet,** daß die Wandergeschwindigkeit der elektrischen Oberflächenwellen sich periodisch ändert und/oder stehende Wellen erzeugt werden.

5. Oberflächenstruktur nach einem der erwähnten Ansprüche, **dadurch gekennzeichnet**, daß die den Elektroden aufgelagerten Isolier-Schichten (23;43,45;53) verlustbehaftete Dielektrika sind, die die Elektroden zumindest teilweise bedecken.

6. Oberflächenstruktur nach Anspruch 5, **dadurch gekennzeichnet,** daß die den Elektroden aufgelagerten Isolier-Schichten (23;53;43,45) aus mehreren Lagen bestehen und/oder mikrostrukturiert sind.

7. Oberflächenstruktur nach einem der Ansprüche 1 bis 6, **dadurch gekennzeichnet,** daß zwischen den Elektrodenstreifen ein anderes dielektrisches Material streifen- oder domänenartig angeordnet ist.

8. Oberflächenstruktur nach einem der Ansprüche 1 bis 7, **dadurch gekennzeichnet,** daß die Elektroden (11;22) streifenförmig oder punktförmig (17b,17b',17b''; 18b,18b',18b''; 19b) ausgebildet sind und im Vergleich zur Elektrodendimension (Breite oder Durchmesser) makroskopische Flächen bedecken ("Elektrodenareale").

9. Oberflächenstruktur nach einem der Ansprüche 1 bis 8, **dadurch gekennzeichnet,** daß - zueinander isoliert (43) - mehrere Elektrodenareale (42,44) übereinander angeordnet sind.

10. Oberflächenstruktur nach einem der Ansprüche 1 bis 9, **dadurch gekennzeichnet,** daß die Elektrodenstreifen senkrecht zur Oberfläche gewellt oder gefaltet sind.

11. Oberflächenstruktur nach einem der Ansprüche 1 bis 10, **dadurch gekennzeichnet,** daß das Substrat (21,41,51) Silizium, Glas, Plastik, Keramik oder flexibles Folien-Material ist.

12. Oberflächenstruktur nach einem der Ansprüche 1 bis 11, **dadurch gekennzeichnet,** daß durch Aussteuerung einzelner Elektroden (22a,22b,22c, 22d; 42a,42b...; 44a,44b...; 52a,52b...) oder Elektrodengruppen in lokal begrenzten Bereichen Adhäsionsmuster mit einem Auflösungsraster der doppelten Elektrodenbreite erzeugt werden.

13. Oberflächenstruktur nach einem der Ansprüche 1 bis 12, **dadurch gekennzeichnet,** daß sie mindestens teilweise auf Rohren, Test- oder Reaktionsräumen, Schläuchen, Kanälen, Sensoroberflächen, Implantatoberflächen oder Lagergefäßen aufgebracht ist.

14. Oberflächenstruktur nach einem der Ansprüche 1 bis 13, **dadurch gekennzeichnet,** daß sie durch Kanäle oder Poren oder sonstige Verbindungen, die die angrenzenden Volumenphasen hydrodynamisch, osmotisch oder elektrisch zueinander in Kontakt bringen, in regelmäßiger oder unregelmäßiger Anordnung durchbrochen ist.

15. Oberflächenstruktur nach einem der Ansprüche 1 bis 14, **dadurch gekennzeichnet,** daß die Elektroden durch elektronische Bauelemente, wie Transistoren oder Dioden, partiell oder vollständig gebildet sind oder angesteuert werden.

16. Oberflächenstruktur nach einem der vorhergehenden Ansprüche, **dadurch gekennzeichnet,**
(a) daß die Signale sich im Voltbereich (0.1V über 0.5V bis 1.5V) für dielektrisch bedeckte Elektrodenareale (42,43,44) befinden;
(b) daß die Signale im unteren Voltbereich (0.01V über 0.05V bis 0.25V) für unbedeckte (blanke) Elektrodenareale (17b,17b',18b) liegen.

17. Oberflächenstruktur nach einem der vorhergehenden Ansprüche, **dadurch gekennzeichnet,** daß die Isolierschicht (23;43,45;53)
(a) eine Dicke (d) im Sub-Mikrometerbereich (von molekularer Dicke bis µm-Dicke) aufweist; und/oder
(b) biokompatibel ist.

18. Oberflächenstruktur nach einem der vorhergehenden Ansprüche, **dadurch gekennzeichnet,** daß die dielektrische Schicht (23;43,45) aus einem in der Halbleitertechnik verwendeten Werkstoff gebildet wird, insbesondere aus SiO₂, SiC, Si₃N₄, Bariumtitanat, Tantaloxyd oder einem Abdecklack.

19. Oberflächenstruktur nach einem der vorhergehenden Ansprüche, **dadurch gekennzeichnet,** daß der Submicronbereich sich von etwa 1µm abwärts erstreckt, insbesondere deutlich unterhalb von 1µm liegt.

20. Oberflächenstruktur nach einem der vorhergehenden Ansprüche, **dadurch gekennzeichnet,** daß die Isolier-Schicht (23;43,45)
(a) eine mechanische Trennschicht, eine elektrische Isolierschicht oder eine Kombination davon ist; und/oder
(b) eine dielektrische Schicht mit insbesondere hohem Dielektrizitäts-Koeffizienten ist.

21. Oberflächenstruktur nach einem der vorhergehenden Ansprüche, **dadurch gekennzeichnet,** bei der die Zuleitungen (17a,17a', 18a,19a) zu den - zur Suspension hin blanken - Elektroden (17b, 17b',18b,19b) im Substrat (21) eingebettet sind.

22. Verwendung der Oberflächenstruktur nach einem der vorhergehenden Ansprüche in einer oder für eine physiologische(n) Lösung hoher Leitfähigkeit, insbesondere Nährlösung.

23. Verwendung der Oberflächenstruktur nach einem der Ansprüche 1 bis 21 als Biosensor.

24. Verwendung der Oberflächenstruktur nach einem der Ansprüche 1 bis 21 zur Stabilisierung von pH-Gradienten in wässrigen oder leitfähigen Lösungen.

## Claims

1. A controllable adhesion surface structure with electrodes (11; 52; 22; 42, 44; 17b, 18b, 19b) on a substrate (21, 41, 51) for use with an aqueous or conductive solution and the microscopic and submicroscopic particles or cells corpuscles) suspended in it, in which surface structure
(a) the electrodes (11; 52; 22; 42, 44; 17b, 18b 19b) have a width and a separation in the submicron region and can be activated in groups by high frequency periodic signals which are phase shifted with respect to each other or pulsed (12 to 15; 34, 33; 17a, 18a, 19a),
(b) so as to decouple the wandering and/or standing electric surface waves resulting therefrom - especially over the insulating layers covering the electrodes (23; 53; 43, 45) - in the aqueous or conductive solution in the neighbourhood of the surface and to control the adhesion relationships in a direction somewhat perpendicular to the substrate surface.

2. A surface structure according to Claim 1, **characterised in that** the wandering electric surface waves generated in each case by at least three electrodes (22a, 22b, 22c, 22d) are periodically reversed and/or run towards each other or away from each other.

3. A surface structure according to Claim 1 or Claim 2, **characterised in that** the wandering electric surface waves generated in each case by at least three electrodes (22a, 22b, 22c) have differing speeds of wandering, that the electrodes (22; 42, 44; 52) of the groups are controlled with different periodic signals in frequency.

4. A surface structure according to one of the foregoing claims, **characterised in that** the speed of wandering of the electric surface waves changes periodically and/or standing waves are generated.

5. A surface structure according to one of the foregoing claims, **characterised in that** the insulating layers (23; 43, 45; 53) covering the electrodes are lossy dielectrics, which at least partly cover the electrodes.

6. A surface structure according to Claim 5, **characterised in that** insulating layers (23; 53; 43, 45) covering the electrodes comprise several layers and/or are microstructured.

7. A surface structure according to one of the Claims 1 to 6, **characterised in that** a different dielectric material is arranged in strips or domains between the electrode strips.

8. A surface structure according to one of the Claims 1 to 7, **characterised in that** the electrodes (11; 22) are formed in strips or in points (17b, 17b', 17b''; 18b, 18b', 18b''; 19b) and in comparison with the electrode dimension (breadth or diameter) cover macroscopic surfaces ("electrode arrays").

9. A surface structure according to one of the claims 1 to 8, **characterised in that** - isolated from each other - several electrode arrays (42, 44) are arranged over each other.

10. A surface structure according to one of the claims 1 to 9, **characterised in that** the electrode strips are waved or folded perpendicularly to the surface.

11. A surface structure according to one of the claims 1 to 10, **characterised in that** the substrate (21, 41, 51) is silicon, glass, plastics, ceramic or flexible foil material.

12. A surface structure according to one of the claims 1 to 11, **characterised in that**, by the activation of individual electrodes (22a, 22b, 22c, 22d; 42a, 42b ...; 44a, 44b ...; 52a, 52b ...) or electrode groups in local limited regions, adhesion models with a resolution grid of double the electrode width are created.

13. A surface structure according to one of the claims 1 to 12, **characterised in that** it is at least partly applied to tubes, test or reaction spaces, sleeves, channels, sensor surfaces, implant surfaces or storage vessels.

14. A surface structure according to one of the claims 1 to 13, **characterised in that** it is broken through in a regular or irregular arrangement by channels or pores or other links, which bring the adjacent volume phases hydrodynamically, osmotically or electrically in contact with each other.

15. A surface structure according to one of the claims 1 to 14, **characterised in that** the electrodes are formed or controlled partially or wholly by electronic components, such as transistors or diodes.

16. A surface structure according to one of the foregoing claims, **characterised in that,**
(a) the signals are in the voltage range (0.1V through 0.5V to 1.5V) for dielectric covered electrode arrays (42, 43, 44);
(b) the signals lie in the lower voltage range (0.01V through 0.05V to 0.25V) for uncovered (blank) electrode arrays (17b, 17b', 18b).

17. A surface structure according to one of the foregoing claims, **characterised in that** the insulating layer (23; 43, 45; 53)
(a) has a thickness (d) in the submicrometer range (from molecular thickness up to µm thickness); and/or
(b) is biocompatible.

18. A surface structure according to one of the foregoing claims, **characterised in that** the dielectric layer (23; 43, 45) is formed from a material used in semiconductor technology, especially from SiO₂, SiC, Si₃N₄, barium titanate, tantalum oxide or a covering lacquer.

19. A surface structure according to one of the foregoing claims, **characterised in** that the submicron range extends from some 1µm downwards, especially significantly below 1µm.

20. A surface structure according to one of the foregoing claims, **characterised in** that the insulating layer (23; 43, 45)
(a) is a mechanical separating layer, an electrical insulating layer or a combination thereof; and/or
(b) a dielectric layer with especially a high dielectric coefficient.

21. A surface structure according to one of the foregoing claims, **characterised in** that the feed wires (17a, 17a', 18a, 19a) to the - towards the suspension - blank electrodes (17b, 17b', 18b, 19b) are embedded in the substrate (21).

22. Application of the surface structure according to one of the foregoing claims in a or for a physiological solution of high conductivity, in particular a nutrient solution.

23. Application of the surface structure according to one of the claims 1 to 21 as a bio-sensor.

24. Application of the surface structure according to one of the claims 1 to 21 for the stabilisation of pH gradients in aqueous or conductive solutions.

## Revendications

1. Structure de surface à adhésion réglable munie d'électrodes (11; 52; 22; 42, 44; 17b, 18b, 19b) sur un substrat (21, 41, 51) pour un usage avec une solution aqueuse ou conductrice et dans laquelle sont mises en suspension des particules ou cellules microscopiques et sous-microscopiques, structure de surface dans laquelle :
(a) les électrodes (11; 52; 22; 42, 44; 17b, 18b, 19b) ont une largeur et une distance de l'ordre des sous-microns et peuvent être alimentées (12 à 15; 34, 33; 17a, 18a, 19a) par des signaux périodiques de hautes fréquences décalés en phase mutuellement ou pulsés en groupes,
(b) pour déclencher les ondes de surface électriques migratoires ou stationnaires qui en résultent - en particulier via les couches isolantes (23; 53; 43, 45) recouvrant les électrodes - dans la solution aqueuse ou conductrice proche de la surface et commander le comportement d'adhésion dans une direction plus ou moins perpendiculaire à la surface du substrat.

2. Structure de surface selon la revendication 1, caractérisée en ce que les ondes de surface électriques migratoires produites par, respectivement, au moins trois électrodes (22a, 22b, 22c, 22d) changent périodiquement de direction de migration et/ou se rapprochent ou s'écartent l'une de l'autre.

3. Structure de surface selon la revendication 1 ou 2, caractérisée en ce que les ondes de surface électriques migratoires produites par, respectivement, au moins trois électrodes (22a, 22b, 22c) possèdent différentes vitesses de migration du fait que les électrodes (22a, 22b, 22c) des groupes sont commandées par des signaux périodiques de différentes fréquences.

4. Structure de surface selon l'une quelconque des revendications précédentes, caractérisée en ce que la vitesse de migration des ondes de surface électriques varie périodiquement et/ou en ce que des ondes stationnaires sont produites.

5. Structure de surface selon l'une quelconque des revendications précédentes, caractérisée en ce que les couches isolantes (23; 43, 45; 53) appliquées sur les électrodes sont des diélectriques à pertes qui recouvrent au moins en partie les électrodes.

6. Structure de surface selon la revendication 5, caractérisée en ce que les couches isolantes (23; 53; 43, 45) appliquées sur les électrodes sont constituées de plusieurs couches et/ou ont une microstructure.

7. Structure de surface selon l'une quelconque des revendications 1 à 6, caractérisée en ce qu'un autre matériau diélectrique est agencé, configuré en bande ou en domaine, entre les bandes d'électrodes.

8. Structure de surface selon l'une quelconque des revendications 1 à 7, caractérisée en ce que les électrodes (11; 22) se présentent sous la forme de bandes ou de points (17b, 17b', 17b''; 18b, 18b', 18b''; 19b) et couvrent des surfaces macroscopiques ("surfaces d'électrodes") par rapport à la dimension (largeur ou diamètre) des électrodes.

9. Structure de surface selon l'une quelconque des revendications 1 à 8, caractérisée en ce que plusieurs surfaces d'électrodes (42, 44) - isolées les unes des autres (43) - sont agencées l'une sur l'autre.

10. Structure de surface selon l'une quelconque des revendications 1 à 9, caractérisée en ce que les bandes d'électrodes sont ondulées ou repliées perpendiculairement à la surface.

11. Structure de surface selon l'une quelconque des revendications 1 à 10, caractérisée en ce que le substrat (21, 41, 51) est formé de silicium, de verre, de matière plastique, de céramique ou d'un matériau flexible en feuille.

12. Structure de surface selon l'une quelconque des revendications 1 à 11, caractérisée en ce que, par commande d'électrodes individuelles (22a, 22b, 22c, 22d; 42a, 42b, ...; 44a, 44b, ...; 52a, 52b, ...) ou de groupes d'électrodes dans des zones délimitées localement, on produit des modèles d'adhésion avec une trame de définition d'une largeur double de celle des électrodes.

13. Structure de surface selon l'une quelconque des revendications 1 à 12, caractérisée en ce qu'elle est appliquée en moins en partie sur des tubes, des zones d'essai ou de réaction, des tubes flexibles, des canaux, des surfaces de capteurs, des surfaces d'implants ou des récipients de stockage.

14. Structure de surface selon l'une quelconque des revendications 1 à 13, caractérisée en ce qu'elle est traversée, dans un agencement régulier ou irrégulier, par des canaux ou des pores ou d'autres connexions qui mettent en contact mutuel les phases volumiques adjacentes par voie hydrodynamique, osmotique ou électrique.

15. Structure de surface selon l'une quelconque des revendications 1 à 14, caractérisée en ce que les électrodes sont formées ou commandées en partie ou en totalité par des composants électroniques, tels que des transistors ou des diodes.

16. Structure de surface selon l'une quelconque des revendications précédentes, caractérisée en ce que :
(a) les signaux se situent dans la plage de voltage (0,1 V à 1,5 V en passant par 0,5 V) correspondant à des surfaces d'électrodes (42, 43, 44) revêtues de diélectrique, et
(b) les signaux se situent dans la plage de voltage inférieure (0,01 V à 0,25 V en passant par 0,05 V) correspondant à des surfaces d'électrodes non revêtues (nues) (17b, 17b', 18b).

17. Structure de surface selon l'une quelconque des revendications précédentes, caractérisée en ce que la couche isolante (23; 43, 45; 53) :
(a) a une épaisseur (d) dans la plage des sous-microns (d'une épaisseur moléculaire à une épaisseur en µm), et/ou
(b) est biocompatible.

18. Structure de surface selon l'une quelconque des revendications précédentes, caractérisée en ce que la couche diélectrique (23; 43, 45) est formée d'un matériau utilisé dans la technique des semi-conducteurs, en particulier du SiO₂, du SiC, du Si₃N₄, du titanate de baryum, de l'oxyde de tantale ou un vernis de revêtement.

19. Structure de surface selon l'une quelconque des revendications précédentes, caractérisée en ce que la plage sous-micronique s'étend d'environ 1 µm vers le bas, en particulier nettement en dessous de 1 µm.

20. Structure de surface selon l'une quelconque des revendications précédentes, caractérisée en ce que la couche isolante (23; 43, 45) :
(a) est une couche de séparation mécanique, une couche isolante électrique ou une combinaison des deux, et
(b) est une couche diélectrique présentant en particulier un coefficient de diélectricité élevé.

21. Structure de surface selon l'une quelconque des revendications précédentes, caractérisée en ce que les lignes d'alimentation (17a, 17a', 18a, 19a) menant aux électrodes (17b, 17b', 18b, 19b) - nues vis-à-vis de la suspension - sont noyées dans le substrat (21).

22. Utilisation de la structure de surface selon l'une quelconque des revendications précédentes dans ou pour une solution physiologique de conductivité élevée, en particulier un bouillon de culture.

23. Utilisation de la structure de surface selon l'une quelconque des revendications 1 à 21 comme biocapteur.

24. Utilisation de la structure de surface selon l'une quelconque des revendications 1 à 21 pour stabiliser des gradients de pH dans des solutions aqueuses ou conductrices.
